# FASCICULE DE BREVET EUROPEEN

(11) **EP 2 444 036 B1**
(45) Date de publication et mention de la délivrance du brevet: **20.04.2016**
(21) Numéro de dépôt: 11185819.7
(22) Date de dépôt: 19.10.2011
(51) Int. Cl.: A61F 5/01

(54) **Orthèse de cheville**
Knöchelorthese
Ankle orthosis

(30) Priorité: 20.10.2010 FR 1058561
(43) Date de publication de la demande: 25.04.2012
(73) Titulaire: RICHARD FRERES, 42530 Saint Genest Lerpt (FR)
(72) Inventeur: Calet, Angélique, 42150 La Ricamarie (FR); Faure, Didier, 43140 Saint Didier en Velay (FR); Gautier, Thomas, 69005 Lyon (FR); Richard, Dominique, 42110 Aurec/Loire (FR); Sandhof, Marc, 56579 Rengsdorf (DE)
(74) Mandataire: Delorme, Nicolas

(56) Documents cités:
- EP-A1- 0 619 102
- WO-A1-94/05236
- DE-B3-102007 049 953
- DE-U1- 9 205 791
- US-A- 5 657 767
- US-A1- 2009 192 428

## Description

La présente invention concerne une orthèse évolutive stabilisatrice de cheville selon la revendication 1.

Une entorse est une lésion d'un ou plusieurs ligaments causée par un choc ou un mouvement contraire au cours duquel les ligaments se trouvent dans une position qui dépasse leur amplitude normale.

La gravité de la lésion est variable et peut prendre la forme d'un étirement des ligaments, de la déchirure de quelques ligaments jusqu'à une rupture totale des ligaments.

L'entorse de la cheville concerne les ligaments latéraux internes et externes mais également les ligaments du médio pied.

Outre l'immobilisation absolue avec plâtres ou résines dont la mise en place est complexe et dont le port est très contraignant, cette pathologie peut être traitée par la mise en place de bandages élastiques, de type strapping, qui présentent de nombreux inconvénients.

Ces bandages doivent être posés par du personnel spécialisé pour éviter un effet garrot et pour éviter des points de compression douloureux au niveau de l'entorse. De plus, ils doivent être renouvelés régulièrement.

Une entorse de cheville peut être également traitée par le port d'une orthèse stabilisatrice de cheville. Cette dernière a une fonction antalgique ainsi qu'une fonction d'immobilisation relative des mouvements de supination et de pronation, sans blocage du mouvement de flexion du pied dans le but de favoriser la cicatrisation ligamentaire.

Il est connu du document WO 94/05236 une orthèse de cheville avec une partie supérieure et une partie de pied, reliées l'une à l'autre par une ou plusieurs connections latérales élastiques qui s'étendent essentiellement depuis une zone du pied située à proximité du talon, dans le prolongement de la partie inférieure de la jambe, en vue de limiter le mouvement de la cheville.

Il est également connu du document US 2009/192428 une bande enveloppante réglable pour le traitement de l'aponévrosite plantaire.

Les orthèses connues ont une action essentiellement anti supination et agissent essentiellement sur la partie arrière du pied ; elles ont donc principalement une action qui favorise la réparation des ligaments latéraux.

En revanche, les orthèses actuelles n'ont que peu d'action sur la partie intermédiaire du pied dit medio pied.

Or, il s'agit d'une région riche en ligaments dont la cicatrisation est mal prise en compte par les orthèses connues.

Les orthèses connues autorisent ainsi des mouvements du pied délétères pour une bonne cicatrisation.

Un but de l'invention est notamment de proposer une orthèse qui favorise la réparation des ligaments du médio pied.

Un autre but de l'invention est de proposer une orthèse qui soit d'une utilisation facile et d'un port confortable.

L'invention concerne une orthèse stabilisatrice de cheville destinée à une immobilisation fonctionnelle d'une zone tibio tarsienne d'une cheville comprenant :
- une chevillière en matière textile élastique conformée de manière anatomique de façon à s'étendre entre une portion inférieure de jambe et une portion de pied, capable d'englober le pied depuis la tête des métatarses jusqu'au talon et jusqu'à la partie inférieure de la jambe, la chevillière comprenant une face médiale I et une face latérale II et étant équipée de moyens de stabilisation de la partie arrière du pied, et
- un module d'avant pied stabilisateur comprenant :
   - (i) un anneau destiné à entourer l'avant pied, pourvu d'une plateforme latéro métatarsienne destinée à venir en appui contre l'arête du côté latérale du pied, et
   - (ii) une sangle anti tiroir dont une première extrémité est connectée à la plateforme latéro métatarsienne et s'étend depuis la plateforme latéro métatarsienne, sous la partie sous métatarsienne du pied, suit la face latérale II de la chevillière, entoure la partie arrière du pied jusqu'à la face médiale I de la chevillière sur laquelle la seconde extrémité de la sangle anti tiroir vient se fixer.

Un a pport essentiel de l'invention est de permettre une immobilisation relative mais surtout fonctionnelle dans la direction antéro postérieure du pied.

Dans une forme de réalisation préférentielle de l'invention, le module d'avant pied comprend, de plus, une sangle anti supination s'étendant depuis la plateforme suivant la chevillière par-dessus la zone de coup de pied jusqu'à la face latérale de la chevillière.

Cette disposition s'avère particulièrement avantageuse puisque le module d'avant pied se combine à la chevillière pour une action anti supination.

L'orthèse selon l'invention permet donc une prise en charge initiale de la pathologie et une adaptation à l'évolution clinique du patient.

Selon d'autres dispositions de l'invention :
- l'anneau comprend deux branches qui viennent se connecter l'une sur l'autre au niveau de la partie métatarsienne du pied,
- le module d'avant pied est indépendant de la chevillière,
- la plateforme latéro métatarsienne comprend un insert en matière souple,
- la chevillière présente une structure tubulaire destinée à être chaussée sur un pied et à englober le pied depuis la tête de métatarses jusqu'au talon et la partie inférieure de la jambe,
- la chevillière présente une fente postérieure,
- la chevillière comprend deux poches obturées par des moyens de fermeture dans chacune desquelles est insérée une coque rigide,
- la chevillière est munie d'une sangle de jambe inférieure et d'une sangle de jambe supérieure, les sangles de jambe inférieure et supérieure entourant la jambe selon des sens opposés,
- la chevillière présente une zone talonnière et une zone de pli de cheville constituée d'un textile dont l'extensibilité est supérieure à celle du textile constituant la chevillière,
- la chevillière présente une sangle de talon qui s'étend depuis de face médiale I de la chevillière vers la face latérale II en passant sous le talon,
- la sangle de réglage de largeur présente une sangle élastique supplémentaire disposée dans son prolongement.

Pour sa bonne compréhension, l'invention est décrite en référence au dessin ci annexé représentant, à titre d'exemple non limitatif, une forme de réalisation d'une orthèse selon celle-ci.

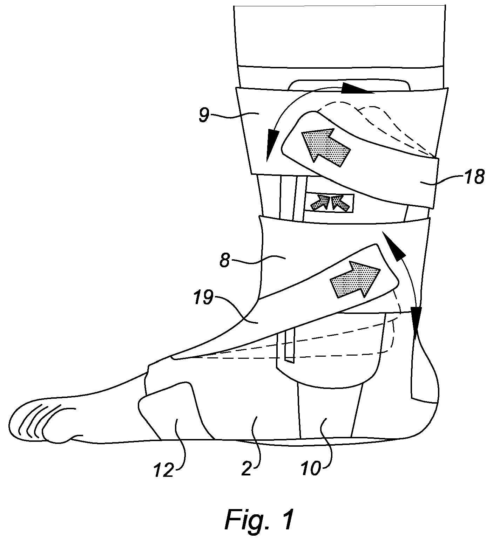

Figure 1 montre la face médiale de l'orthèse stabilisatrice de cheville en position sur la zone tibio-tarsienne d'une jambe,
Figure 2 montre les deux sous-ensembles de l'orthèse selon l'invention, à savoir une chevillière et un module d'avant pied,
Figure 3 montre la chevillière en position sur la zone tibio-tarsienne d'une jambe,
Figure 4 montre la chevillière en vue de devant,
Figure 5 montre la chevillière en cours de mise en position,
Figure 6 montre le module d'avant pied lors de sa jonction avec la chevillière,

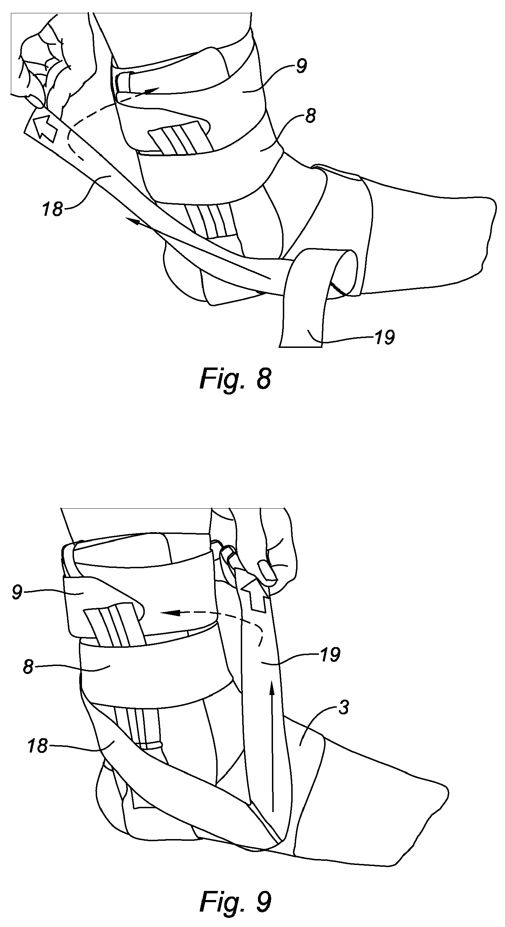

Figures 7, 8 et 9 montrent la mise en place du module d'avant pied sur la chevillière.

Dans la suite, le terme « médial » associé à la référence I définit le côté ou la face de l'orthèse qui est le plus proche de l'axe medio sagittal du corps tandis que le terme « latéral » associé à la référence II définit le côté ou la face de l'orthèse qui est le plus éloigné de l'axe medio sagittal.

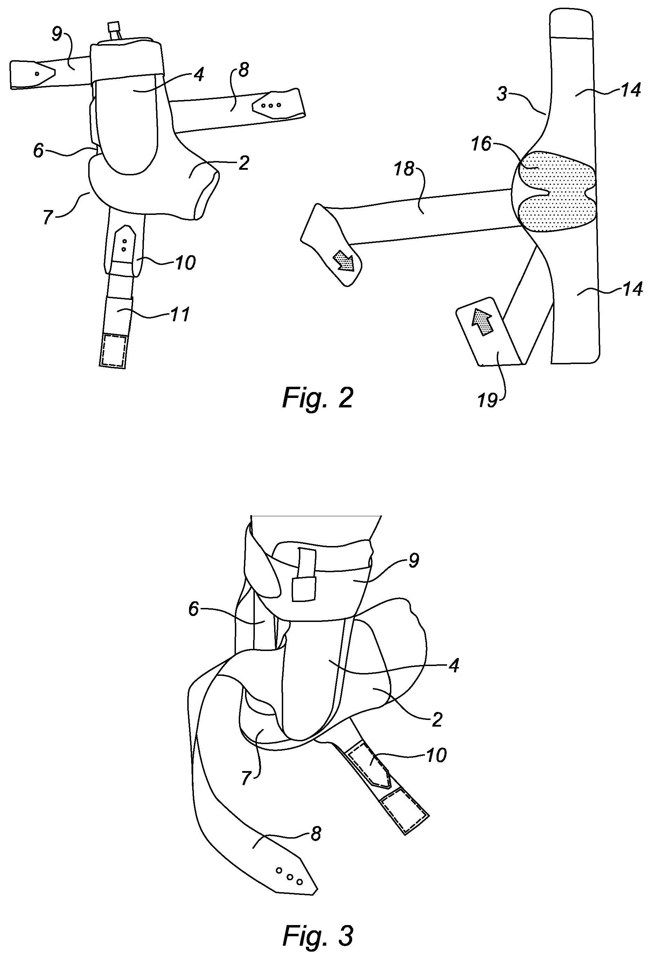

On pourra se reporter tout d'abord à la figure 2 pour apprécier la structure de l'orthèse 1 stabilisatrice de cheville selon l'invention.

Cette orthèse 1 stabilisatrice de cheville comprend essentiellement deux sous-ensembles, à savoir une chevillière 2 et un module d'avant pied 3 dont on va pouvoir apprécier la manière avec laquelle il coopère avec la chevillière 2.

La chevillière 2, telle qu'on peut la voir à la figure 2, présente une structure tubulaire et est conformée de manière anatomique pour pouvoir être chaussée sur un pied. La chevillière 2 englobe le pied depuis la tête du métatarse jusqu'au talon et à la partie inférieure de la jambe. De préférence, la chevillière 2 est réalisée par tricotage.

On peut noter que la chevillière 2 présente deux poches 4 latérales qui peuvent être chacune fermées, comme le montre la figure 2, par une fermeture à glissière. Les fermetures à glissière donnent accès à l'intérieur de la poche 4 dans laquelle une coque (non représentée) peut être insérée. Par coque, on entend un élément allongé généralement en matière plastique semi rigide qui a, pour effet, de rigidifier les parois latérales de la chevillière 2 augmentant ainsi la stabilisation de la partie arrière du pied.

Bien que cela n'apparaisse pas sur la figure 2, la chevillière 2 présente au droit de chacune des poches 4, un capitonnage intérieur pour éviter toute blessure. II peut également être prévu que les poches présentent une doublure dans une matière plastique, de type -polypropylène ou similaire, qui contribue à rigidifier les parois latérales de la chevillière 2 lorsque les coques sont retirées.

La chevillière 2 présente, par ailleurs, une fente postérieure 6 qui s'étend jusqu'à une zone talonnière 7.

La chevillière 2 a pour fonction principale de stabiliser la partie arrière du pied. Cette fonction est remplie par la raideur du textile dans lequel elle est coupée combinée aux coques amovibles et - de manière plus marginale - aux doublures plastiques.

La chevillière 2 est aidée dans cette fonction par plusieurs sangles 8, 9, 10 qui, dans l'exemple représenté, sont au nombre de trois.

Ainsi, la chevillière 2 possède une sangle de jambe inférieure 8 et une sangle de jambe supérieure 9. Ces deux sangles viennent entourer la jambe dans des sens opposés. En pratique, chacune des sangles inférieure 8 et supérieure 9 est fixée sur un des bords de la fente postérieure 6 et entoure la chevillière 2 en passant sur la fente postérieure 6.

Comme on le voit sur la figure 2, la chevillière 2 est également dotée d'une sangle de talon 10. La sangle de talon 10 est fixée sur l'un des côtés de la chevillière 2 en bas de la poche 4. La sangle de talon 10 passe sous le talon pour venir se fixer sur la face latérale opposée.

Il est possible, comme cela est montré sur la figure 2, de doter la sangle de talon 10, dans son prolongement, d'une sangle élastique supplémentaire 11. La fonction de la sangle élastique supplémentaire 11 est d'autoriser le mouvement de supination du pied, tout en appliquant une force visant à ramener le pied en position de sécurité.

La fixation de l'extrémité libre des sangles 8, 9, 10, 11 se fait sur la chevillière 2 par des moyens classiques, à savoir des zones de tissu auto agrippant à boucles et crochets.

Le module d'avant pied 3 présente une structure tout à fait différente de celle de la chevillière 2 dans la mesure où il comprend un anneau 12 ouvert en deux branches 14. Les deux branches 14 peuvent être réunies par un élément de jonction auto agrippant à boucles et crochets.

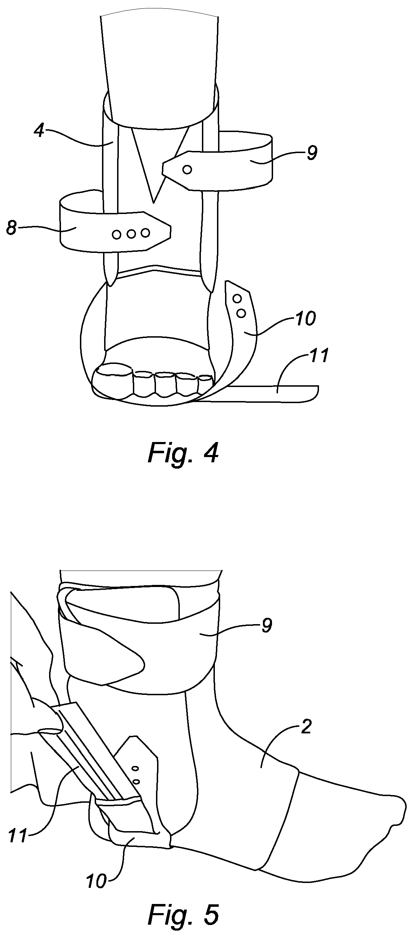

Un point important du module d'avant pied 3 est la présence d'une plateforme 16 latéro métatarsienne sensiblement au milieu de l'anneau 12. La plateforme 16 latéro métatarsienne peut être constituée, comme cela est montré à la figure 5, par un insert en matière souple. L'insert peut être constitué d'une matière synthétique de type PVC, d'un textile raidi ou encore de cuir.

Le module d'avant pied comprend deux sangles 18 et 19 qui sont fixées à la plateforme 16 latéro métatarsienne. Il s'agit d'une sangle dite sangle anti tiroir 18 qui est orientée de manière sensiblement perpendiculaire aux deux branches 14 formant l'anneau et une sangle dite sangle anti supination 19 qui forme un angle aigu avec la branche 14 qui lui est immédiatement adjacente.

La mise en place de l'orthèse 1, selon l'invention, se fait dans une première étape en chaussant la chevillière 2.

La chevillière 2, une fois qu'elle est en position sur le patient, englobe le pied depuis la tête des métatarses jusqu'au talon et jusqu'à la partie inférieure de la jambe.

La figure 4 montre la manière avec laquelle les différentes sangles 8, 9, 10, 11 sont mises en place.

Les deux sangles inférieure 8 et supérieure 9 de jambe viennent ceinturer la jambe en sens opposé, tandis que la sangle de talon 10 vient s'étendre depuis un côté de la chevillière 2 jusqu'au côté opposé en passant au dessous du talon. La sangle élastique supplémentaire 11 est ensuite positionnée pour appliquer une force tendant à ramener le pied en position de sécurité. La figure 5 illustre la mise en place de la sangle de talon 10.

On peut remarquer que la chevillière 2 est d'une mise en place aisée puisqu'elle est dotée d'une fente postérieure 6.

Il est également à noter que la zone talonnière 7 peut être réalisée préférentiellement dans un textile élastique tricoté qui est plus extensible que le textile qui constitue l'ensemble de la chevillière 2 qui est de préférence raide ; cette disposition a un effet très bénéfique pour le confort puisqu'il permet d'éviter la création d'un effet de garrot au niveau du pli de la cheville.

Le confort de cette chevillière 2 est donc grandement amélioré.

La chevillière 2 seule, comme on peut la voir par exemple à la figure 5, a un effet qui se concentre essentiellement sur les ligaments latéraux.

En revanche, la chevillière 2 seule n'est pas en mesure de stabiliser parfaitement l'articulation tibio tarsienne dans les directions antérieure et postérieure, si bien qu'un mouvement antéro postérieur dit en tiroir reste possible. Ce type de mouvement étant extrêmement délétère pour la cicatrisation des ligaments de la zone du médio pied.

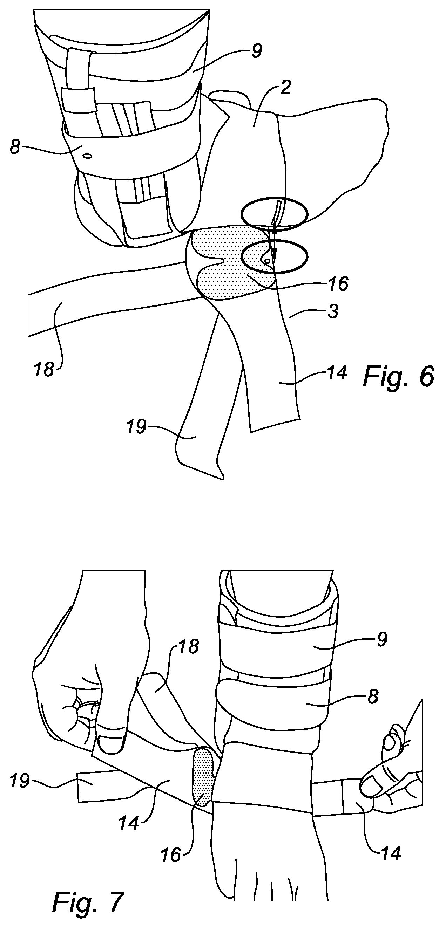

Le module d'avant pied 3, comme on peut le voir par exemple sur la figure 6, est associé à la chevillière 2. Pour cela, la plateforme 16 latéro métatarsienne est positionnée contre l'arête du côté latéral du pied. Dans cette position, la plateforme 16 latéro métatarsienne est bien supportée par le patient car la plateforme 16 prend une forme sensiblement de tuile pour suivre le contour anatomique de la zone latéro métatarsienne.

Un élément de fixation de type textile auto agrippant à boucles et crochets peut être prévu pour fixer le module d'avant pied 3 sur la chevillière 2. Les deux branches 14 de l'anneau sont réunies sur la partie du coup de pied, puis les sangles anti tiroir 18 et anti supination 19 sont mises en place.

La sangle anti tiroir 18 s'étend depuis la partie sous métatarsienne du pied, suit la face latérale de la chevillière 2, puis contourne la partie arrière du pied en venant entourer la zone du tendon d'Achille jusqu'à la face médiale I de la chevillière 2 où l'extrémité libre de la sangle anti tiroir vient se fixer.

La sangle anti tiroir a donc une action extrêmement novatrice dans la mesure où elle vient interdire le mouvement du pied dans la direction antéro postérieure.

L'action du module d'avant pied 3 se prolonge par la sangle anti supination 19 qui part également de la plateforme 16 sous latéro métatarsienne et qui s'étend depuis la face latérale II de la chevillière 2 par-dessus la zone de coup de pied jusqu'à la face médiale I de la chevillière 2 où l'extrémité de la sangle anti supination vient se fixer par accrochage auto agrippant.

La sangle anti supination 19 vient ainsi compléter l'action de stabilisation anti supination qui est déjà en partie fournie par la chevillière 2.

Compte tenu des efforts importants qui sont exercés par la sangle anti tiroir 18 et par la sangle anti supination 19, on comprend que le module d'avant pied 3 comprend un insert en matière plastique relativement rigide. Cet insert, en matière plastique, permet d'absorber les efforts de traction importants qui s'exercent au travers de la sangle anti tiroir.

Dans l'exemple montré, le module d'avant pied 3 est amovible de la chevillière 2, ce qui permet de s'adapter à l'évolution de la pathologie.

Dans une autre forme de réalisation, il pourrait être prévu d'intégrer à demeure le module d'avant pied 3 à une chevillière 2.

Bien entendu, l'invention ne se limite pas à la forme de réalisation décrite ci-dessus mais elle en embrasse au contraire toutes les variantes de réalisation.

## Revendications

1. Orthèse (1) stabilisatrice de cheville destinée à immobilisation fonctionnelle d'une zone tibio tarsienne d'une cheville comprenant :
- une chevillière (2) en matière textile élastique conformée de manière anatomique de façon à s'étendre entre une portion inférieure de jambe et une portion de pied, capable d'englober le pied depuis la tête des métatarses jusqu'au talon et jusqu'à la partie inférieure de la jambe, la chevillière (2) présentant une face médiale I et une face latérale II et étant équipée de moyens de stabilisation de la partie arrière du pied, et
**caractérisée en ce que** l'orthèse comprend aussi
- un module d'avant pied (3) stabilisateur comprenant
o un anneau (12) destiné à entourer l'avant pied, pourvu d'une plateforme (16) latéro métatarsienne destinée à venir en appui contre l'arête du côté latéral du pied et
o une sangle anti tiroir (18) dont une première extrémité est connectée à la plateforme (16) latéro métatarsienne et s'étend depuis la plateforme (16) latéro métatarsienne, sous la partie sous métatarsienne du pied, suit la face latérale II de la chevillière (2), entoure la partie arrière du pied jusqu'à la face médiale I de la chevillière (2) sur laquelle la seconde extrémité de la sangle anti tiroir (19) vient se fixer.

2. Orthèse (1) selon la revendication 1, **caractérisée en ce que** le module d'avant pied (3) comprend de plus une sangle anti supination (19) s'étendant depuis la plateforme (16) latéro métatarsienne suivant la chevillière (2) par-dessus la zone de coup de pied jusqu'à la face médiale I de la chevillière (2).

3. Orthèse (1) selon la revendication 1 ou la revendication 2, **caractérisée en ce que** l'anneau (12) comprend deux branches (14) d'anneau qui viennent se connecter l'une sur l'autre au niveau de la partie métatarsienne du pied.

4. Orthèse (1) selon l'une des revendications 1 à 3, **caractérisée en ce que** le module d'avant pied est indépendant de la chevillière (2).

5. Orthèse (1) selon l'une des revendications 1 à 4, **caractérisée en ce que** la plateforme (16) latéro métatarsienne comprend un insert en matière plastique souple.

6. Orthèse (1) selon l'une des revendications 1 à 4, **caractérisée en ce que** la chevillière (2) présente une structure tubulaire destinée à être chaussée sur un pied et à englober le pied depuis la tête de métatarses jusqu'au talon et la partie inférieure de la jambe.

7. Orthèse (1) selon l'une des revendications 1 à 6, **caractérisée en ce que** la chevillière (2) présente une fente postérieure (6).

8. Orthèse (1) selon l'une des revendications 1 à 7, **caractérisée en ce que** la chevillière (2) comprend deux poches (4) obturées par des moyens de fermeture dans chacune desquelles est insérée une coque rigide.

9. Orthèse (1) selon l'une des revendications 1 à 8, **caractérisée en ce que** la chevillière (2) est munie d'une sangle de jambe inférieure (8) et d'une sangle de jambe supérieure (9), les sangles de jambe inférieure (8) et supérieure (9) entourant la jambe selon des sens opposés.

10. Orthèse (1) selon l'une des revendications 1 à 9, **caractérisée en ce que** la chevillière (2) présente une zone talonnière (7) et une zone de pli de cheville constituées d'un textile dont l'extensibilité est supérieure à celle du textile constituant la chevillière (2).

11. Orthèse (1) selon l'une des revendications 1 à 10, **caractérisée en ce que** la chevillière (2) présente une sangle de talon (10) qui s'étend de la face médiale I de la chevillière (2) vers la face latérale II de la chevillière (2) en passant sous le talon.

12. Orthèse (1) selon la revendication 11, **caractérisée en ce que** la sangle de talon (10) est munie d'une sangle élastique supplémentaire (11) disposée dans son prolongement.

## Patentansprüche

1. Stabilisierende Knöchelorthese (1), die zum funktionalen Stillstellen einer Sprunggelenkzone eines Knöchels bestimmt ist, die Folgendes umfasst:
- eine Knöchelbandage (2) aus elastischem Textilmaterial, die auf anatomische Art derart ausgebildet ist, dass sie sich zwischen einem unteren Beinabschnitt und einem Fußabschnitt erstreckt, die fähig ist, den Fuß vom Metatarsalienkopf bis zur Ferse und bis zum unteren Teil des Beins zu umschließen, wobei die Knöchelbandage (2) eine mediale Seite I und eine seitliche Seite II aufweist und mit Stabilisierungsmitteln des hinteren Teils des Fußes ausgestattet ist, und
**dadurch gekennzeichnet, dass** die Orthese auch
- ein stabilisierendes Vorderfußmodul (3) umfasst, das Folgendes umfasst
-- einen Ring (12), der dazu bestimmt ist, den Vorderfuß zu umgeben, der mit einer seitlichen Metatarsal-Plattform (16) versehen ist, die dazu bestimmt ist, gegen eine Kante der seitlichen Seite des Fußes zum Aufliegen zukommen, und
-- einen Rutschschutzgurt (18), von dem ein erstes Ende mit der seitlichen Metatarsal-Plattform (16) verbunden ist und sich von der seitlichen Metatarsal-Plattform (16) unter dem submetatarsalen Teil des Fußes erstreckt, der seitlichen Seite II der Knöchelbandage (2) folgt, den hinteren Teil des Fußes bis zur medialen Seite I der Knöchelbandage (2) umgibt, auf der das zweite Ende des Rutschschutzgurts (19) befestigt wird.

2. Orthese (1) nach Anspruch 1, **dadurch gekennzeichnet, dass** das Vorderfußmodul (3) außerdem einen Supinationsschutzgurt (19) umfasst, der sich von der seitlichen Metatarsal-Plattform (16) entlang der Knöchelbandage (2) über die Fußristzone bis zur medialen Seite I der Knöchelbandage (2) erstreckt.

3. Orthese (1) nach Anspruch 1 oder Anspruch 2, **dadurch gekennzeichnet, dass** der Ring (12) zwei Ringschenkel (14) umfasst, die sich aufeinander im Bereich des Metatarsalteils des Fußes verbinden.

4. Orthese (1) nach einem der Ansprüche 1 bis 3, **dadurch gekennzeichnet, dass** das Vorderfußmodul von der Knöchelbandage (2) unabhängig ist.

5. Orthese (1) nach einem der Ansprüche 1 bis 4, **dadurch gekennzeichnet, dass** die seitliche Metatarsal-Plattform (16) einen Einsatz aus biegsamem Plastik umfasst.

6. Orthese (1) nach einem der Ansprüche 1 bis 4, **dadurch gekennzeichnet, dass** die Knöchelbandage (2) eine röhrenförmige Struktur aufweist, die dazu bestimmt ist, auf einem Fuß angezogen zu werden und den Fuß von dem Metatarsalienkopf bis zur Ferse und den unteren Teil des Beins einzuschließen.

7. Orthese (1) nach einem der Ansprüche 1 bis 6, **dadurch gekennzeichnet, dass** die Knöchelbandage (2) einen hinteren Schlitz (6) aufweist.

8. Orthese (1) nach einem der Ansprüche 1 bis 7, **dadurch gekennzeichnet, dass** die Knöchelbandage (2) zwei Taschen (4) umfasst, die durch Verschlussmittel verschlossen sind, in welche jeweils eine starre Schale eingefügt ist.

9. Orthese (1) nach einem der Ansprüche 1 bis 8, **dadurch gekennzeichnet, dass** die Knöchelbandage (2) mit einem unteren Beingurt (8) und einem oberen Beingurt (9) versehen ist, wobei der untere Beingurt (8) und der obere Beingurt (9) das Bein in entgegengesetzte Richtungen umgeben.

10. Orthese (1) nach einem der Ansprüche 1 bis 9, **dadurch gekennzeichnet, dass** die Knöchelbandage (2) eine Fersenzone (7) und eine Knöchelfaltenzone aufweist, die aus einem Textil bestehen, dessen Dehnbarkeit größer ist als die des Textils, das die Knöchelbandage (2) bildet.

11. Orthese (1) nach einem der Ansprüche 1 bis 10, **dadurch gekennzeichnet, dass** die Knöchelbandage (2) einen Fersengurt (10) aufweist, der sich von der medialen Seite I der Knöchelbandage (2) zu der seitlichen Seite II der Knöchelbandage (2) erstreckt, indem er unter der Ferse durchgeht.

12. Orthese (1) nach Anspruch 11, **dadurch gekennzeichnet, dass** der Fersengurt (10) mit einem zusätzlichen elastischen Gurt (11), der in seiner Verlängerung angeordnet ist, versehen ist.

## Claims

1. An ankle stabilizing orthosis (1) for functional immobilization of a tibiotarsal area of an ankle comprising:
- an anklet (2) made of elastic textile material anatomically shaped so as to extend between a leg lower portion and a foot portion, capable of embracing the foot from the head of the metatarsi to the heel and to the lower part of the leg, the anklet (2) having a medial face I and a lateral face II and being equipped with stabilizing means of the rear part of the foot, and
**characterized in that** the orthosis also comprises
- a stabilizing forefoot module (3) comprising
o a ring (12) intended to surround the forefoot, provided with a latero-metatarsal platform (16) intended to bear against the ridge of the lateral side of the foot and
o an anti-slide strap (18) whose one first end is connected to the latero-metatarsal platform (16) and extends from the latero-metatarsal platform (16), under the sub-metatarsal part of the foot, follows the lateral face II of the anklet (2), surrounds the rear part of the foot to the medial face I of the anklet (2) on which the second end of the anti-slide strap (19) is secured.

2. The orthosis (1) according to claim 1, **characterized in that** the forefoot module (3) further comprises an anti-supination strap (19) extending from the latero-metatarsal platform (16) along the anklet (2) over the instep area to the medial face I of the anklet (2).

3. The orthosis (1) according to claim 1 or 2, **characterized in that** the ring (12) comprises two ring branches (14) which are connected on each other at the metatarsal part of the foot.

4. The orthosis (1) according to any of claims 1 to 3, **characterized in that** the forefoot module is independent of the anklet (2).

5. The orthosis (1) according to any of claims 1 to 4, **characterized in that** the latero-metatarsal platform (16) comprises an insert made of flexible plastic material.

6. The orthosis (1) according to any of claims 1 to 4, **characterized in that** the anklet (2) has a tubular structure intended to be put on a foot and to embrace the foot from the head of the metatarsi to the heel and the lower part of the leg.

7. The orthosis (1) according to any of claims 1 to 6, **characterized in that** the anklet (2) has a posterior slit (6).

8. The orthosis (1) according to any of claims 1 to 7, **characterized in that** the anklet (2) comprises two pockets (4) closed by closing means in each of which a rigid shell is inserted.

9. The orthosis (1) according to any of claims 1 to 8, **characterized in that** the anklet (2) is provided with a lower leg strap (8) and an upper leg strap (9), the lower (8) and upper (9) leg straps surrounding the leg along opposite directions.

10. The orthosis (1) according to any of claims 1 to 9, **characterized in that** the anklet (2) has a heel area (7) and an ankle fold line constituted of a textile whose extensibility is greater than that of the textile constituting the anklet (2).

11. The orthosis (1) according to any of claims 1 to 10, **characterized in that** the anklet (2) has a heel strap (10) which extends from the medial face I of the anklet (2) to the lateral face II of the anklet (2) by passing under the heel.

12. The orthosis (1) according to claim 11, **characterized in that** the heel strap (10) is provided with an additional elastic strap (11) disposed in its extension.
